# EUROPEAN PATENT APPLICATION

(11) **EP 2 223 670 A1**
(43) Date of publication of application: **01.09.2010**
(21) Application number: 08858321.6
(22) Date of filing: 15.08.2008
(51) Int. Cl.: A61F 13/15, A61F 13/472, A61F 13/511

(54) **ABSORBENT ARTICLE AND METHOD OF PRODUCING THE SAME**

(30) Priority: 06.12.2007 JP 2007316410
(71) Applicant: Unicharm Corporation, Ehime 799-0111 (JP)
(72) Inventor: KASHIWAGI, Masahiro, Kanonji-shi Kagawa 769-1602 (JP); OKADA, Saori, Kanonji-shi Kagawa 769-1602 (JP)
(74) Representative: Murgatroyd, Susan Elizabeth
(86) International application number: PCT/JP2008/064631
(87) International publication number: WO 2009/072325

(57) **Abstract**

The present invention aims to provide an absorbent article adapted to maintain a desired flexibility of the topsheet and to prevent the article from becoming glossy.
A sanitary napkin 1 comprises a topsheet 2 defining an inner side facing the wearer's skin, an outer side facing the wearer's garment, a liquid-absorbent structure 4 interposed between these top- and backsheets 2, 3, and a cushion sheet 5 interposed between the liquid-absorbent structure 4 and the topsheet 2. The topsheet 2 is formed with a plurality of pores 6 assuring liquid-pervious property thereof. The pores 6 are distributed over an entire area of the topsheet 2 and extending through the topsheet 2 in its thickness direction. The topsheet 2 is debossed to form a plurality of concavities 13 directed toward the side of the cushion sheet 5. The concavities 13 respectively have substantially circular shapes and are distributed over an entire area of the topsheet 2 substantially at regular intervals debossing to form the concavities 13 is carried out at a temperature lower than a fusion point of the topsheet 2.

## Description

### TECHNICAL FIELD

The present invention relates to absorbent articles and particularly to absorbent articles such as sanitary napkins.

### RELATED ART

Sanitary napkins adapted to come in contact with the wearer's skin with soft touch and to prevent a topsheet facing the wearer's skin from being stuck fast to the skin has well known, for example, from JP 1996-504607W. The sanitary napkin disclosed in JP 1996-504607W comprises a topsheet defining an inner side facing the wearer's garment, a backsheet defining an outer side facing the napkin wearer's clothes, an absorbent structure interposed between these top- and backsheets, and a cushion sheet interposed between the absorbent structure and the topsheet. The cushion sheet is formed to be bulky so that bodily fluids may smoothly spread and a comfortable touch may be assured for the wearer's skin. The cushion sheet and the topsheet are joined to each other by heat sealing technique. The joint of these two sheets presents concavities directed from the side of the topsheet toward the side of the cushion sheet and a plurality of these concavities are arranged substantially at regular intervals. By joining the cushion sheet and the topsheet in this fashion, there is no fear that the topsheet might be peeled off from the cushion sheet and be stuck fast to the napkin wearer's skin.
PATENT DOCUMENT 1: 1996-504607W

### DISCLOSURE OF THE INVENTION

### PROBLEM TO BE SOLVED BY THE INVENTION

Joining the topsheet to the cushion sheet by use of heat sealing technique causes the topsheet to be fused in the joint region and the sheet partially fused in this manner has a stiffness increased, disadvantageously leading to increased irritation of the wearer' s skin due to the increased stiffness. The partially fused region of the topsheet inevitably becomes glossy and such gloss evolves uncomfortable phenomenon such that the topsheet might be stuck fast to the wearer's skin and often causes stuffiness and/or a feeling of discomfort.

It is also contemplated to bond the topsheet to the cushion sheet by means of adhesive instead of heat sealing technique. However, a large quantity of adhesive is required for such bonding since the material fibers must be loosely entangled to make the cushion sheet bulky. The topsheet is formed with a plurality of pores in order to ensure its liquid-pervious property and the adhesive possibly seeps from these pores if a large quantity of adhesive is used.

In view of the problem as has been described above, the present invention provides an absorbent article adapted to maintain a desired flexibility of the topsheet and to prevent the article from becoming glossy.

### MEASURE TO SOLVE THE PROBLEM

According to the present invention, there is provided an absorbent article having an inner side facing the wearer's skin, an outer side facing the wearer's garment, a liquid-pervious topsheet defining the side facing the wearer's skin and formed with a plurality of pores, a liquid-impervious backsheet defining the side facing the wearer's garment, a liquid-absorbent structure interposed between the top- and backsheets and a cushion sheet interposed between the liquid-absorbent structure and the topsheet.

The improvement according to the present invention is **characterized in that** the topsheet is bonded to the cushion sheet by means of adhesive and formed with a plurality of concavities directed from the side facing the wearer's skin toward the cushion sheet by means of debossing without forming any fusion portion due to heat.

According to one preferred embodiment, the topsheet is formed from a thermoplastic resin and the debossing is carried out at a temperature lower than a fusion point of the thermoplastic resin.

According to another preferred embodiment, the adhesive is a pressure-sensitive adhesive.

According to the present invention, there is provided a method for making an absorbent article having an inner side facing the wearer's skin, an outer side facing the wearer's garment, a liquid-pervious topsheet defining the inner side facing the wearer' s skin and provided with a plurality of pores, a backsheet defining the outer side facing the wearer's garment and a cushion sheet provided on the side of the topsheet facing the wearer's garment.

According to the present invention comprising the steps of coating the cushion sheet with adhesive, laminating the topsheet on the cushion sheet coated with the adhesive, the topsheet and the cushion sheet laminated together are debossed, and joining the topsheet and the backsheet to each other, wherein the debossing is carried out by feeing the laminate to pass through debossing apparatus including an debossing roll, the debossing roll being maintained at a temperature lower than a fusion point of the topsheet and formed with a plurality of convex die regions, and the convex die regions being pressed from the side of said topsheet toward the cushion sheet.

### EFFECT OF THE INVENTION

The topsheet and the cushion sheet are bonded together by means of adhesive and the topsheet is debossed to form a plurality of the concavities directed toward the cushion sheet. In this way, the area over which the topsheet is put in contact with the cushion sheet can be enlarged. The contact area may be enlarged to enhance bond strength between the topsheet and the cushion sheet correspondingly. In consequence, it is not required to use a large quantity of adhesive.
The cushion is pressurized at regions in which the concavities are formed and thereby the topsheet and the cushion sheet are firmly bonded without requiring use of the heat sealing technique. Therefore the topsheet should not become stiff due to heat. In this way, the topsheet can be maintained flexible and possible irritation of the wearer's skin can be effectively restricted. Furthermore, it is also possible to prevent the topsheet from becoming glossy since the heat sealing technique is not used.

The topsheet is formed from a thermoplastic resin and the process of debossing the topsheet to form the concavities is carried out at a temperature lower than a fusion point of this thermoplastic resin. In consequence, the process of debossing the topsheet to form the concavities can be reliably achieved without fusing the topsheet.

The pressure-sensitive adhesive is used as the adhesive and therefore it is unnecessary to heat the topsheet for adhesion. Thus undesired fusion of the topsheet can be reliably avoided. Particularly in the regions of the concavities, the topsheet and the cushion sheet are pressurized so as to enhance the bond strength between these two sheets.

The topsheet is subjected to the process of debossing after coating of the adhesive between the topsheet and the cushion sheet and thereby the concavities being stuck from the topsheet in the cushion sheet can be formed. As an advantageous result, the contact area between the topsheet and the cushion sheet can be enlarged and, at the same time, these two sheets can be firmly bonded together.

### BRIEF DESCRIPTION OF THE DRAWINGS

[FIG. 1] Fig. 1 is a plan view of sanitary napkin.
[FIG. 2] Fig. 2 is a sectional view taken along the line II-II in Fig. 1.
[FIG. 3] Fig. 3 is a scale-enlarged view showing a substantial part in Fig. 1.
[FIG. 4] Fig. 4 is a diagram schematically illustrating a method for making the sanitary napkin.
[FIG. 5] Fig. 5 is a sectional view taken along the line V-V in Fig. 3.

### IDENTIFICATION OF REFERENCE NUMERALS USED IN THE DRAWINGS

- 1: sanitary napkin
- 2: topsheet
- 3: backsheet
- 4: liquid-absorbent structure
- 5: cushion sheet
- 6: pores
- 13: convexities
- 15: debossing apparatus
- 16: adhesive coater
- 17: adhesive
- 21: heat debossing roll
- 23: convexity die region

### IDENTIFICATION OF REFERENCE NUMERALS USED IN THE DRAWINGS

Detail of the present invention will be more fully understood from the description of a sanitary napkin as one of typical embodiments of the absorbent article given hereunder with reference to the accompanying drawings.

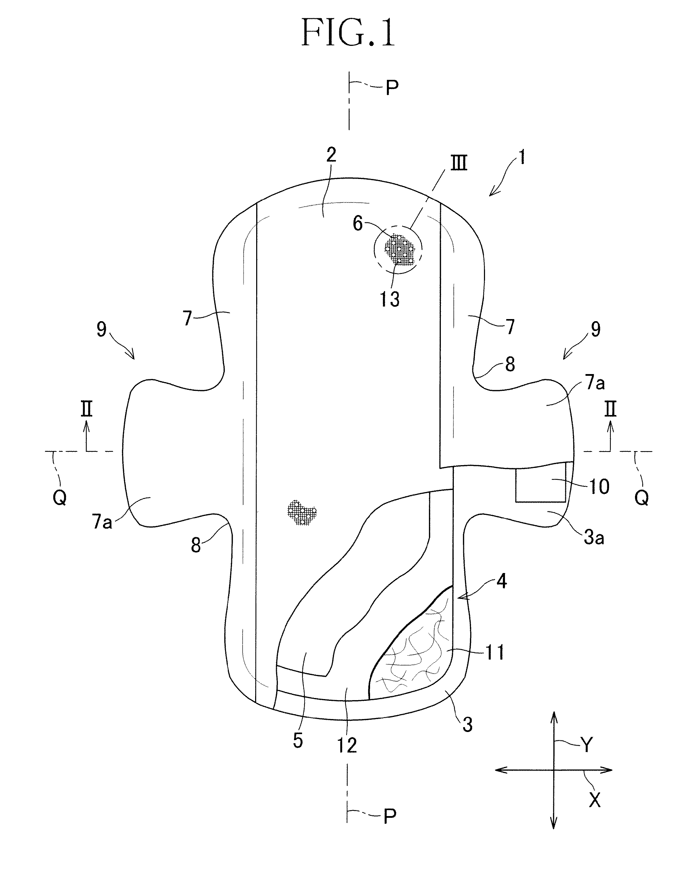

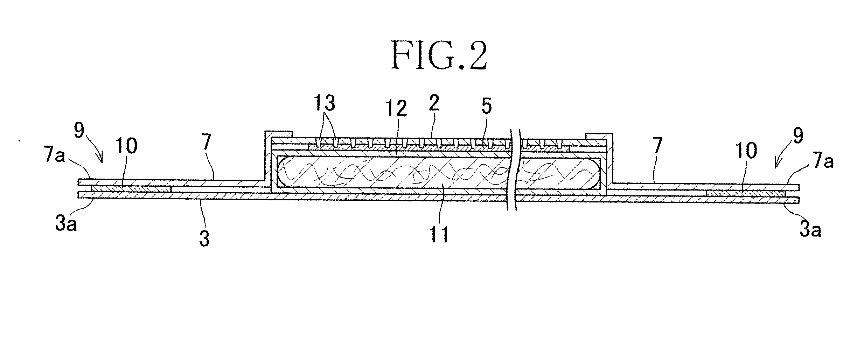

Fig. 1 is a plan view of the sanitary napkin 1 and Fig. 2 is a sectional view taken along the line II-II in Fig. 1. As will be apparent from Fig. 1, the sanitary napkin 1 has a transverse direction X with respect to the napkin wearer's body, a longitudinal direction Y orthogonal to the transverse direction X, a longitudinal center line P-P bisecting a dimension of the sanitary napkin 1 as measured in the transverse direction X and a transverse center line Q-Q bisecting a dimension of the sanitary napkin 1 as measured in the longitudinal direction Y. The sanitary napkin 1 comprises a topsheet 2 defining an inner side facing the wearer's skin, a backsheet 3 defining an outer side facing the wearer's garment, a liquid-absorbent structure 4 interposed between these top- and backsheets 2, 3 and a cushion sheet 5 interposed between the liquid-absorbent structure 4 and the topsheet 2.

The topsheet 2 is a rectangular sheet extending in the longitudinal direction Y and formed from a thermoplastic film. The topsheet 2 is formed with a plurality of pores 6 serving to assure liquid-permeability of the topsheet 2. These pores 6 are distributed over its entire area and extend through the topsheet 2 in its thickness direction. The topsheet 2 is provided on its side edges opposed to each other in the transverse direction X with side sheets 7 respectively extending in the longitudinal direction Y of the topsheet 2. The opposite side edges of the topsheet 2 and the associated side sheets 7 are joined together by means of adhesive or the other means (not shown). Specifically, the inner side facing the wearer's skin is defined by this assembly of these topsheet 2 and the side sheets 7 joined together in this manner. The side sheets 7 may be formed, for example, from a liquid-pervious fibrous nonwoven fabric and extend, in the vicinity of the transverse center line Q-Q, outward in the transverse direction X.

The backsheet 3 defining the outer side has substantially the same shape as the assembly of the topsheet 2 and the side sheets 7 defining the inner side facing the wearer's skin and is formed from a liquid-impervious film. The assembly of the topsheet 2 and the side sheets 7 is joined to the backsheet 3 by means of adhesive or the other means (not shown) along an outer periphery of the backsheet 3 to integrate them. Portions 7a of the side sheets 7 extending outward in the transverse direction X cooperate with portions 3a of the backsheet 3 opposed to the respective portions 7a to form flaps 9. To immobilize the sanitary napkin 1 on the wearer's shorts, these flaps 9 may be folded around peripheral edges of the respective leg-openings of the shorts. Between the portions 7a of the respective side sheets 7 and the respective portions 3a of the backsheet 3, there are provided reinforcing sheets 10 serving to enhance stiffness of the flaps 9 and thereby to facilitate the sanitary napkin to be put on the wearer's body. These portions 3a, 7a and the reinforcing sheets 10 are joined together by means of adhesive (not shown).

The liquid-absorbent structure 4 interposed between the top- and backsheets 2, 3 comprises a liquid-absorbent core 11 which comprises, in turn, a mixture of fluff pulp and super-absorbent polymer particles, and a liquid-absorbent and liquid-spreadable sheet 12 such as tissue paper with which the liquid-absorbent core 11 is wrapped.

As has previously described, the substantially rectangular cushion sheet 5 extending in the longitudinal direction Y is sandwiched between the liquid-absorbent structure 4 and the topsheet 2. The cushion sheet 5 is formed from a liquid-absorbent fibrous nonwoven fabric so as to assure soft touch for the napkin wearer and at the same time to guide bodily fluids to the liquid-absorbent structure 4. To ensure a desired degree of bodily fluid permeability, this cushion sheet 5 is formed from loosely entangled composite fibers composed of polypropylene as the core and polyethylene as the sheath. Alternatively, it is possible to obtain the desired cushion sheet by use of hydrophilic fibers such as cellulose fibers or hydrophobic fibers such as polyolefin or polyester incorporated or coated with an appropriate modifying agent to make it hydrophilic. To meet another requirement for the cushion sheet 5, i.e., to assure comfortably soft touch experienced by the sanitary napkin wearer, the fibers may be loosely entangled to make the sanitary napkin 1 bulky in its thickness direction and thereby to assure high resiliency.
The cushion sheet 5 and the topsheet 2 laminated therewith are bonded to each other by means of adhesive (not shown).

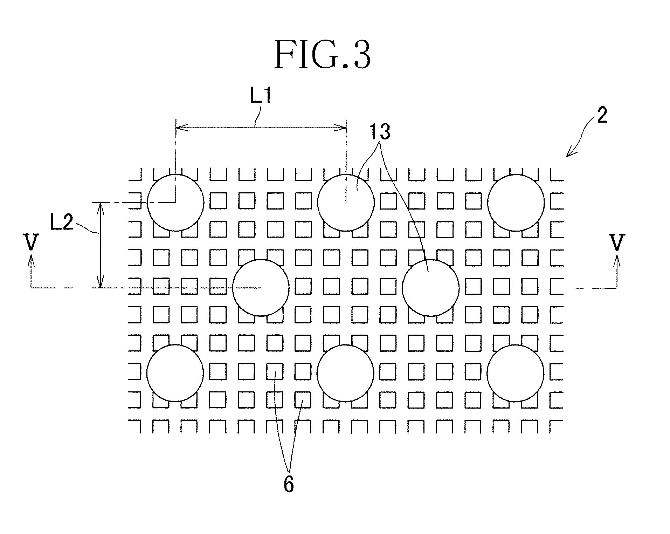

The topsheet 2 is deboss-treated to form a plurality of concavities directed to the side of the cushion sheet 5. These concavities 13 are substantially circle-shaped and distributed over the entire area of the topsheet 2 substantially at regular intervals. Fig. 3 is a scale-enlarged diagram illustrating a relevant part of the topsheet 2 shown in Fig. 1. The concavity 13 has a diameter of about 1.2 mm and a plurality of rows each containing these concavities 13 arranged at regular intervals L1 of about 6 mm in the transverse direction X are arranged in the longitudinal direction Y. Row to row distance L2 is about 3 mm and the concavities 13 contained in each of rows are staggered from the concavities 13 contained in the adjacent row by about 3 mm in the transverse direction X. In other words, the concavities 13 are arranged in a stagger pattern defined by a pitch of 3 mm and an angle of 45°. An area ratio of these concavities 13 to the topsheet 2 is 6.28%.

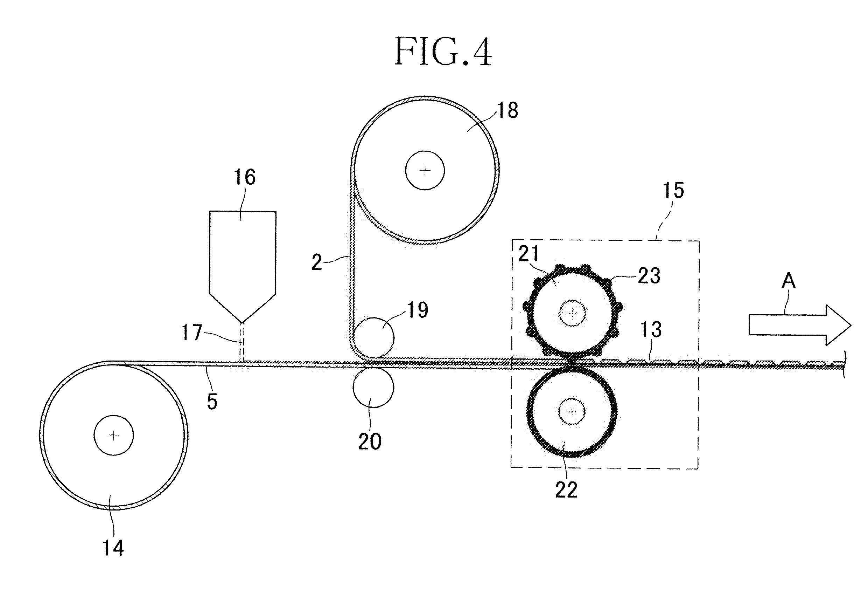

As illustrated in Fig. 4, the topsheet 2 is debossed by a pair of debossing or embossing (hereinafter referred to as "debossing") rolls and thereby formed with the concavities 13 directed from the side of the topsheet 2 toward the side of the cushion sheet 5. More specifically, the cushion sheet 5 is from a cushion sheet feed roll 14 and transported by a conveyor belt (not shown) toward a heat debossing apparatus 15 in a direction indicated by an arrow A. The cushion sheet 5 fed in this manner is coated by an adhesive coater 16 with adhesive 17. Hot melt pressure-sensitive adhesive of about 7 g/m² is used as the adhesive and coated in a spiral pattern. The cushion sheet 5 coated with the adhesive is further transported in the direction of the arrow A.

The topsheet 2 fed from a topsheet feed roll 18 is laminated on the surface of the cushion sheet 5 coated with the adhesive 17. The cushion sheet 5 and the topsheet 2 laminated one upon another are transported so as to pass through a pair of pressure rolls 19, 20 in which the cushion sheet 5 and the topsheet 2 are pressurized and joined together. The laminate of these cushion sheet 5 and topsheet 2 is now transported to the heat debossing apparatus 15 and guided through a heat debossing roll 21 and an anvil roll 22. While the heat debossing roll 21 is formed on its peripheral surface with convex die regions 23, the anvil roll 22 is formed on its peripheral surface with none of irregularities. In the course of passing through between these rolls 21, 22, the laminate is debossed by the convex die regions 23 from the side of the topsheet 2 toward the side of the cushion sheet 5 and the topsheet 2 is formed with the concavities 13. While the laminate is pressurized by the pressure rolls 19, 20 after coated with the adhesive 17 so far as this embodiment is concerned, it is possible to pressurize the laminate by the heat debossing roll 21 and the anvil roll 22 without following the step of adhesive coating. Preferably, in this case, the anvil roll 22 also is formed on its peripheral surface with irregularities, specifically, concavities adapted to be engaged with the convex die regions 23 on the heat debossing roll 21. This is for the reason that, in this way, a bonding effect of the adhesive can be enhanced particularly in the regions of engagement between the convexities on the heat debossing roll 21 and the corresponding concavities formed on the anvil roll 22. In the vicinity of these convexities and concavities also, the bonding effect can be enhanced. In the case of the anvil roll 22 having no irregularities, the bonding effect can be relied only on the convex die regions 23 on the heat debossing roll 21 and it is appreciated that the topsheet 2 and the cushion sheet 5 might peel off from each other in non-bonded region.

A debossing temperature of the heat debossing roll 21 is set to the temperature at which the topsheet 2 is not molten. The topsheet 2 is principally made of low density polyethylene having a fusion point in a range of about 90 to 110°C. Considering this, the debossing temperature is set to a temperature at least lower than 90°C, preferably a temperature 40°C or higher but lower than 90°C. The debossing carried out at a temperature about 50°C lower than the fusion point allows the topsheet 2 to be prevented from fusion and ensures that the topsheet 2 can be debossed more reliably than the case in which the debossing is carried out without heating.

The cushion sheet 5, the topsheet 2 and the side sheets 7 joined together define the side facing the sanitary napkin wearer's skin. The liquid-absorbent structure 4 is interposed between the sheets 2, 7 and the backsheet 3 and the sheet 2, 7 and the backsheet 3 are jointed together along respective outer peripheries thereof to form the sanitary napkin 1.

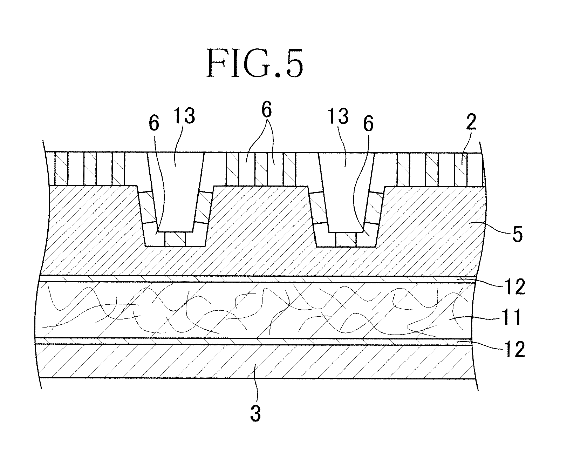

Fig. 5 is a sectional view taken along the line V-V in Fig. 3. As shown, the heat debossed concavities 13 are formed so as to stick in the cushion sheet 5. A total area in which the topsheet 2 is held in contact with the cushion sheet 5 can be enlarged by forming the concavities 13 in the manner as has been describe above and thereby a joining effect of the adhesive (not shown) for these two sheets 2, 5 can be enhanced. The cushion sheet 5 is relatively bulky because of loosely entangled material fibers and the area in which the cushion sheet 5 comes in contact with the topsheet 2 is correspondingly small. Therefore, an adequate joining strength can not be expected from use of the adhesive only and a desired joining strength can be obtained first by forming the concavities 13.

The topsheet 2 and the cushion sheet 5 are bonded to each other using pressure-sensitive adhesive to laminate these two sheets one on another and this laminate is pressurized by the heat debossing rolls to form the concavities 13. In this way, the joining effect can be enhanced. The joining effect can be enhanced by enlarging the contact area and pressurizing the laminate, advantageously leading to reduction of the adhesive to be used. Reduction of the adhesive to be used allows the production cost to be correspondingly reduced and allows stiffness of the topsheet 2 to be prevented from unacceptably increasing due to the presence of the adhesive.

The concavities 13 are debossed at a temperature lower than the fusion point of the topsheet 2 and the topsheet 2 should not be fused in the course of the debossing. In consequence, stiffness of the topsheet 2 can be prevented from increasing due to its fusion and thereby a desired soft touch experienced by the napkin wearer can be maintained. Furthermore, the topsheet should not become glossy due to fusion and therefore the napkin wearer can use the napkin without being reminded of a discomfortable feeling due to luster gloss.

Should the concavities be fused at a temperature close to its fusion point or higher than its fusion point, not only the concavities 13 but also the region in the vicinity thereof will be fused. If such fusion occurs in a wide range, not only the region having unacceptably high stiffness as well as the regions having unacceptably significant luster gloss will be enlarged but also the pores 6 of the topsheet 2 will be collapsed. If the pores 6 are collapsed, the liquid-pervious property of the topsheet 2 will be deteriorated. According to the present invention, the topsheet 2 should not be fused and, in consequence, the pores 6 should not be collapsed. Therefore the topsheet 2 can be maintained to be liquid-pervious.

The concavities are debossed under a mechanical pressure to enhance the effect of the adhesive to join the topsheet 2 and the cushion sheet 5 together. The topsheet 2 may be added with flatting agent such as titanium oxide or barium sulfate to improve further such effect. This is for the reason that the fatting agent is exposed on the surface of the topsheet at the respective concavities 13 as the topsheet 2 is stretched at the respective concavities 13. Exposure of the fatting agent such as titanium oxide leads to the formation of pores in the topsheet 2 and thereby to improvement in the air-permeability of the topsheet 2.

While the concavities 13 are formed at a temperature for debossing in a range of 40 to 90°C according to the present embodiment, the temperature for debossing may be appropriately selected depending on the components constituting the topsheet 2. It is also possible to vary the temperature in relationship with a period for debossing. For example, debossing is carried out at a relatively high temperature, the period for debossing may be reduced to improve the productivity. When the debossing is carried out at a relatively high temperature, it will be required to cool the debossing die regions in order to maintain the initial shapes of these regions and when the debossing is carried out at a relatively low temperature, such step of cooling the debossing die regions may be eliminated.

Regions of the cushion sheet 5 into which the concavities 13 stick have density increased and correspondingly exhibit the liquid-absorbent capacity enhanced in comparison with the remaining region. Consequentially, the concavities 13 function to guide bodily fluid discharged onto the topsheet 2 in positive manner to the liquid-absorbent structure 4 and thereby to prevent bodily fluid from staying on the topsheet 2.

While each of the concavities 13 has a circular shape according to the present embodiment, the shape of the concavity is not limited to such circular shape and may present, for example, square, oval or rhombic shape and these concavities may be irregularly arranged. Each of the concavities 13 preferably has an area in a range of 0.1 to 20 mm². The area of the concavity 13 less than 0.1 mm² may make it difficult to deboss it and, even if the concavity 13 can be formed, a size thereof should not be too small to be appealing. Function of the concavities 13 is not only to enhance the joint between the topsheet 2 and the cushion sheet 5 but also to improve the design flattering effect. The concavity having its area larger than 20 mm² may cause a problem that the outer periphery of the concavity uncomfortably irritates the napkin wearer's skin.

While the area ratio of the concavities 13 to the topsheet 2 is set to 6.28% according to the present embodiment, this area ratio is preferably in a range of about 0.3 to 50%. If the area ratio is less than about 0.3%, the concavities will not be sufficiently appealing and, if the area ratio exceeds about 50%, the outer periphery of the concavity will be enlarged so as to irritate the napkin wearer's skin.
While the concavities 13 are arranged at the pitch of about 3 mm according to the present embodiment, the pitch is preferably in a range of about 3 to 15 mm. The pitch less than about 3 mm may it difficult to peel off the topsheet 2 from the heat debossing roll 21 in the step of heat debossing.

## Claims

1. An absorbent article having an inner side facing the wearer's skin, an outer side facing the wearer's garment, a liquid-pervious topsheet defining said side facing the wearer's skin and formed with a plurality of pores, a liquid-impervious backsheet defining said side facing the wearer's garment, a liquid-absorbent structure interposed between said top- and backsheets and a cushion sheet interposed between said liquid-absorbent structure and said topsheet, said absorbent article being **characterized in that**:
said topsheet is bonded to said cushion sheet by means of adhesive and formed with a plurality of concavities directed from the side facing the wearer' s skin toward said cushion sheet by means of debossing without forming any fusion portion due to heat.

2. The absorbent article as defined by Claim 1, wherein said topsheet is formed from a thermoplastic resin and said debossing is carried out at a temperature lower than a fusion point of said thermoplastic resin.

3. The absorbent article as defined by Claim 1, wherein said adhesive is pressure-sensitive adhesive.

4. A method for making an absorbent article having an inner side facing the wearer's skin, an outer side facing the wearer' s garment, a liquid-pervious topsheet defining said inner side facing the wearer' s skin and provided with a plurality of pores, a backsheet defining said outer side facing the wearer's garment and a cushion sheet provided on the side of said topsheet facing the wearer's garment, said method comprising the steps of:
coating said cushion sheet with adhesive,
laminating said topsheet on said cushion sheet coated with said adhesive,
said topsheet and said cushion sheet laminated together are debossed, and
joining said topsheet and said backsheet to each other,
wherein said debossing is carried out by feeing the laminate to pass through debossing apparatus including an debossing roll, said debossing roll being maintained at a temperature lower than a fusion point of said topsheet and formed with a plurality of convex die regions, and said convex die regions being pressed from the side of said topsheet toward said cushion sheet.
